# EUROPEAN PATENT SPECIFICATION

(11) **EP 3 082 591 B1**
(45) Date of publication and mention of the grant of the patent: **23.08.2023**
(21) Application number: 14873074.0
(22) Date of filing: 19.12.2014
(51) Int. Cl.: A61B 5/389, A61F 2/72, A61F 2/60, A61B 5/11, A61B 5/00, A61F 2/50, A61F 2/68, A61F 2/76

(54) **SYSTEM AND METHOD FOR NEUROMUSCULAR REHABILITATION COMPRISING PREDICTING AGGREGATED MOTIONS**
SYSTEM UND VERFAHREN ZUR NEUROMUSKULÄREN REHABILITATION MIT VORHERSAGE AGGREGIERTER BEWEGUNGEN
SYSTÈME ET PROCÉDÉ DE RÉÉDUCATION NEUROMUSCULAIRE COMPRENANT DES MOUVEMENTS AGRÉGÉS DE PRÉDICTION

(30) Priority: 20.12.2013 SE 1351570
(43) Date of publication of application: 26.10.2016
(73) Proprietor: Integrum AB, 431 37 Mölndal (SE)
(72) Inventor: ORTIZ CATALAN, Max Jair, S-411 18 Göteborg (SE)
(74) Representative: Kransell & Wennborg KB
(86) International application number: PCT/SE2014/051546
(87) International publication number: WO 2015/094112

(56) References cited:
- EP-B1- 1 838 270
- WO-A1-2008/022435
- WO-A1-2013/001358
- WO-A2-2006/086504
- CN-A- 101 667 346
- DE-A1-102010 006 301
- US-A1- 2009 069 898
- US-A1- 2011 152 249
- US-A1- 2013 338 540
- ORTIZ-CATALAN M; ET AL.: 'Treatment of phantom limb pain (PLP) based on augmented reality and gaming controlled by myoelectric pattern recognition: a case study of a chronic PLP patient' FRONTIERS IN NEUROSCIENCE vol. 8, 25 February 2014, XP055353012
- YOUNG A J; ET AL.: 'A comparison of the real-time controllability of pattern recognition to conventional myoelectric control for discrete and simultaneous movements' JOURNAL OF NEUROENGINEERING AND REHABILITATION vol. 11, 10 January 2014, XP021173488
- ORTIZ-CATALAN M; ET AL.: 'Evaluation of classifier topologies for the real-time classification of simultaneous limb motions' 35TH ANNUAL INTERNATIONAL CONFERENCE OF THE IEEE EMBS 03 July 2013, pages 6651 - 6654, XP032489376
- KHONG L M D; ET AL.: 'Multi-layer perceptron training algorithms for pattern recognition of myoelectric signals' THE 2013 BIOMEDICAL ENGINEERING INTERNATIONAL CONFERENCE 23 October 2013, pages 1 - 5, XP032531688
- AL-JUMAILY A; ET AL.: 'Bio-driven system-based virtual reality for prosthetic and rehabilitation systems' SIGNAL, IMAGE AND VIDEO PROCESSING vol. 6, no. 1, 02 September 2010, pages 71 - 84, XP035016614
- LAMOUNIER E ET AL: "On the use of Virtual and Augmented Reality for upper limb prostheses training and simulation", 2010 ANNUAL INTERNATIONAL CONFERENCE OF THE IEEE ENGINEERING IN MEDICINE AND BIOLOGY SOCIETY : (EMBC 2010) ; BUENOS AIRES, ARGENTINA, 31 AUGUST - 4 SEPTEMBER 2010, IEEE, PISCATAWAY, NJ, USA, 31 August 2010 (2010-08-31), pages 2451-2454, XP032108391, DOI: 10.1109/IEMBS.2010.5626370 ISBN: 978-1-4244-4123-5

## Description

### FIELD OF THE INVENTION

The present invention relates to a system and a method for neuromuscular rehabilitation of a patient.

### BACKGROUND OF THE INVENTION

A successful rehabilitation is crucial for the quality of life after a traumatic event such as a limb amputation, which besides depriving a limb, in many cases results in phantom limb pain (PLP). Analogously, patients who have suffered stroke, incomplete spinal cord, and/or other nerve injuries, face neuromuscular rehabilitation challenges to alleviate what otherwise become life-permanent handicaps. PLP is a deteriorating condition commonly suffered by amputees. In fact, studies show that 70%-85% of amputees suffer from PLP. Traditionally, a method known as mirror therapy is used for relieving PLP. In mirror therapy, the motion of a healthy contralateral limb is mapped onto a representation of the missing limb such that the patient visually experiences motor execution in the missing limb by moving both limbs in the same manner.

The conventional mirror therapy method consists in placing a physical mirror in the plane separating the missing limb from the contralateral limb. This very crude type of mirror therapy can be replaced by more sophisticated methods and systems. For example, in the last decade, virtual reality (VR) has been employed as a more sophisticated version of conventional mirror therapy. Despite the advantages of employing VR over a traditional physical mirror, known VR approaches have retained the use of the contralateral limb and are therefore restricted to unilateral amputees. Promotion of motor execution (for example by producing phantom motions) in order to revert cortical reorganization, is the neuroscientific base of the mirror treatments. However, when using the contralateral limb as source of control, the actual effort made by the patient on the affected limb is disregarded, thus hindering the efficacy of the therapy.

One example of a VR based system is disclosed by US8568231 in which an entire virtual limb grossly moves based on motion sensors at the missing limb, rather than sensors on the contralateral limb. However, movements are determined from signals based on position data received from transmitters located at the location of e.g. a missing limb. Thereby, the number of possible motions is rather limited because an intended motion affects an entire virtual extension of the missing limb as a whole, rather than its individual components. A similar work is disclosed in "ReckAR, master's thesis by Morten Bak Kristoffersen, Aalborg University". Another exemplary VR system is disclosed in WO2006/086504.

Additional previous related work is described in "Evaluation of post-processing strategies for simultaneous pattern recognition based myoelectric prosthetic control (Joel Falk-Dahlin, master's thesis from Chalmers University, Sweden)". However, as indicated by the title, this work was related to post-processing of prerecorded and available data.

A further relevant document for understanding the background of the invention is described in "On the use of virtual and augmented reality for upper limb prostheses training and simulation. (2010; Lamounier, E; Lopes, K; Cardoso, A; Andrade, A; Soares, A)"

In view of the above, there is a need for a system and method with improved promotion of motor execution for patients in need of neuromuscular rehabilitation, such as for example related to PLP.

### SUMMARY OF THE INVENTION

In view of the above-mentioned and other drawbacks of the prior art, a general object of the present invention is to provide a system and a method in which a virtual limb, computer games, and/or robotic devices, respond directly to the intention of motor execution decoded from bioelectric signals of the subject, and thus promoting motor control over the affected limb.

The invention is defined by the appended claims.

The present invention is based on the realization that promotion of motor execution enables an improved neuromuscular rehabilitation, and in the case of amputees, a reduction in PLP. Furthermore, the present invention is based on the realization that the motor execution is improved by enabling direct response of a virtual limb, games, and/or robotic devices, controlled via the predicted motions.

In the present context, an affected limb may be a disordered, missing, or an otherwise not fully functional limb. For example, an amputee has a missing limb which in the context of the present invention may be an affected limb. Furthermore, a patient who by any other means may not control a limb normally, for example as a result of incomplete spinal injury or stroke, may have an affected limb. In the present context, intent to move an affected limb refers to an attempt to move for example a malfunctioning limb or a missing limb.

In accordance with the invention, pattern recognition is the recognition of certain patterns in the electric signals acquired from a portion of the patient's body. For example by measuring an electric potential from a portion of a patient's body in the vicinity of the affected limb and predicting the intended motion according to the similarities of previously recorded patterns. Pattern of electric signals are characterized by signal features. The electric signals according to the invention may also be bioelectric signals.

In the present context an individual motion may be flexing of a joint, extending the joint, pronation, supination, open or close hand (in the case where a hand is part of an affected limb, and include several joints), ulnar deviation, radial deviation, or any other motion possible with a limb. The control unit thus extracts features indicative of an intended motion to feed a pattern recognition algorithm. Furthermore, an aggregated motion is the combination of one or more individual motions. For example, to reach for an object and grip the object with a hand, or grabbing an object with a hand and rotating it through supination of the hand, or open a hand while performing pronation of the hand. Furthermore, in the case of hand open/close, all the joints of the fingers are actuated together, thus resulting in aggregated motions (e.g. individual and simultaneous motions). In other words, several joints are performing individual motions at least partly simultaneously. Moreover, with aggregated motions the joints may be actuated individually, thus moving individual portions of the limb, or all together, for example with different speeds and ranges if necessary. Furthermore, an aggregated motion may be a first individual motion followed by a second individual motion.

In accordance with a configuration, a feature may be for example signal variance, median frequency, root mean square, rate of pulse trains, or any other signal feature known in the art. A non-exhaustive list is of features in the time domain of an electrical signal is comprised in the following: mean absolute value, median, standard deviation, variance, waveform length, root mean square (RMS), zero-crossings of the signal, peaks (RMS), peaks mean, mean velocity, slope changes, power, difference absolute mean, max fractal length, fractal dimension, fractal dimension Higuchi, rough entropy, correlation, co-variance, etc. A non-exhaustive list is of features in the frequency domain of an electrical signal is comprised in the following: waveform length, mean, median, peaks mean, peaks median, peaks standard deviation, etc.

In accordance with the invention, neuromuscular rehabilitation comprises condition in which motor execution is required e.g. phantom limb pain, stroke, nerve and/or spinal cord injuries.

According to one embodiment of the invention, at least two aggregated motions of the affected limb may be predicted, wherein the control unit is configured to control the feedback member to perform at least two actions corresponding to the at least two motions.

According to one configuration, the feedback member may be a robotic device. For example, the robotic device may be a remotely controlled vehicle, helicopter, prosthetic device or any other device which may be remotely controlled. A prosthetic device may be a prosthetic arm, leg, hand, foot, etc. Furthermore, the robotic device may comprise a sensor which measures a force applied to the sensor (thereby also to the robotic device). The sensor may send a signal to the control unit which may control an audio, visual, or tactile feedback about the presence or magnitude of the force. For example, the frequency or volume of an audio feedback may depend on the magnitude of the force, or the frequency or strength of the tactile feedback may depend on the magnitude of the force or may reflect the force measure by the force sensor. The force sensor may for example be a force sensor comprising e.g. a piezoelectric element, conductive polymer element or similar.

According to one embodiment of the invention, the system further comprises a display arranged to provide the real-time visual feedback to the patient, the feedback member being a virtual limb, wherein the actions are motions performed by the virtual limb, such that the control unit is configured to: based on the output signals from the performed pattern recognition, control the virtual limb on the display to perform the motions, whereby the motions of the virtual limb are individually and simultaneously controlled by the patient via the intended motions. The virtual limb enhances the feeling for the patient of having a real arm, thus promoting motor execution further. In the present context, real-time may be delayed only by the control unit due to processing of the electric signals and controlling the virtual limb to perform the motions on the display.

According to one embodiment of the invention, the system may comprise an video capturing device arranged to acquire a video stream of the patient, the visual feedback comprises the video stream, wherein the control unit is configured to superimpose the virtual limb onto a predetermined portion of the patient's body in the visual feedback being displayed on the display, the virtual limb corresponding to the affected limb. By using an actual acquired image or video of the patient and superimposing a virtual limb in the image/video the illusion of a restored limb is enhanced. Thus, the patient will see him/herself on the display with a virtual arm in place of the affected or disordered limb. Superimposing a virtual limb into a real image enables an augmented reality. For example, when the patient intends to perform a motion in the affected limb, the virtual limb on the display moves according to the intended motion predicted via pattern recognition.

According to one embodiment of the invention, the virtual limb follows the predetermined portion of the patient's body in the visual feedback being displayed on the display such that the virtual limb remains in an anatomically correct position. In other words, if the patient moves around, the virtual arm stays in the anatomically correct place where the affected (e.g. missing limb) should be. This way, the illusion of a restored limb is further enhanced. According to the invention, the virtual limb follows the predetermined position by allowing the control unit to track, via the camera, fiducial markers arranged on the patient's affected limb in the position where the virtual limb is to be superimposed. A fiducial marker may for example be printed using a conventional printer, or the fiducial marker may be a light-emitting diode. Thereby, the virtual limb is superimposed on the marker as shown in the display, and the virtual limb changes scale and rotation based on the tracking of the marker.

According to one configuration, for pattern recognition, the control unit may be configured to: divide each of the electric signals into signal segments defined by time intervals; extract signal features from the segments; combine the features into a feature vector relating to the motion; and based on the feature vector, predict the intended motion of the affected limb, wherein the features comprise an extracted cardinality of the data elements of the electrical signal. Accordingly, a feature vector may comprise at least one feature wherein one of the features is the cardinality feature. A cardinality of a vector is the number of unique elements of the vector. For example, if a signal is defined by the elements in the vector [1 1 2 3 5] the cardinality of the signals is four. Using the cardinality of the signal in each segment as a feature for pattern recognition, the accuracy of the pattern recognition is increased. As an example, the following is the average class-specific accuracy of a commonly used classifier (Linear Discriminant Analysis, LDA) in the prediction of 11 hand and wrist motions in 20 subjects when feed with the following features:
Cardinality: 90.0%
Zero crossings: 82.1%
Wave length: 82.0%
Root mean square value: 81.8%
Mean absolute value: 80.1%
Sign slope change: 74.7%

As can be seen from the above accuracies of prediction, the prediction accuracy when using the cardinality feature is higher compared to when using the other commonly used features listed above, resulting in an improved pattern recognition and thus prediction of intended motions.

For pattern recognition and thus prediction of an intended motion, a time window is first determined. For example, a time window may be 10-500 milliseconds, such as 200 milliseconds. The signals received by the control unit from the electrodes, one signal per electrode or electrodes pair, is divided or split in intervals of e.g. 200 milliseconds. One such interval is a segment. Thus, in accordance with the invention the electric signal is divided into segments where a segment is a portion of an electric signal defined by a certain time interval. The segments may or may not overlap. Moreover, at least one feature is extracted in a segment for each electrode (i.e. channel). For example, a first feature from the first channel and a second feature from the second channel etc. More than one feature per segment may be extracted and the type of feature extracted from different channels may be the same type of feature. The features extracted in the first segment of each channel are inserted into a vector known as a feature vector. In other words, a vector comprising all the features extracted during a time interval/segment. Algorithms used for pattern recognition may be e.g. supervised, unsupervised, and/or competitive, as well of a statistical nature (e.g. LDA), or biologically inspired (e.g. artificial neural networks, ANN). The algorithms may be arranged in different topologies (e.g. One-Vs. -One). The feature vector characterizes for example a motion.

In one configuration, the signal features are extracted from the time or frequency domain. For example, in the frequency domain one may more clearly identify the effect of fatigue, as fast fibers are not fatigue resistant.

According to one embodiment of the invention, the control unit is configured to, based on the output signals from the pattern recognition, control a video game on the display. A video game is an engaging tool for promoting motor execution since motivation increasing tasks may be implemented. For example, the feeling of completing a new level in a game or beating a competitor gives a patient a feeling of success, and thus a motivation for continuing the game while at the same time exercising motion in the affected limb. A video game may be specifically designed to promote motor execution of a particular degree of freedom. The video game is thus controlled by the patient through his/her intended motion of the affected limb. A video game may for example be a racing game, a fighting game, a platform game, etc.

According to one configuration, the electrodes may be implantable into a patient's body for detecting bioelectrical signals. Using implantable electrodes is useful in case a patient is to control a prosthetic device, or for improved motor execution with direct contact with at least one nerve or muscle. Furthermore, an increased signal strength and signal-to-noise ratio may be possible which facilitates control over a virtual arm, games, or robotic device (e.g. prosthesis). Furthermore, with implantable electrodes a naturally perceived feedback by stimulating the nerves is possible. For example, with the implantable electrodes physiological feedback to the nerves may be provided such that the patient may for example experience the sensation of feeling an object using e.g. a prosthetic device.

Furthermore, the electrodes may be connected via an osseointegrated implant. For example, the implantable electrodes may be connected to the nerves via an osseointegrated implant.

In one embodiment, the visual feedback is in virtual reality. In other words, the entire visual feedback is virtually artificial with no real parts of the patient is included. Thus, a virtual world (not reflecting the real world) is shown on the display.

According to one embodiment of the invention, the electrodes are configured to measure myoelectric signals from the surface of the skin of the patient. This way, an easy manner to measure the (bio)electric signals is achieved by placing the electrodes on the surface of a patient's skin.

Furthermore, said control unit may be configured to, based on said output signals from said pattern recognition, control computer unit input commands. For example, the input commands may be to control the pointer on the computer unit, to press "return" or "space", to open a web-browser, or any other software. Thus, the control unit may convert the signals acquired by the electrodes to input commands for the computer unit. The conversion may for example be made via a look-up table relating features of the acquired signals to input commands.

According to one embodiment of the invention, wherein said plurality of electrodes is a high density electrode array. In other words, the electrodes are placed close to each other such that signals are measured from the surface of the patient's skin from densely located points. This way, signals with sufficient quality may be found in a facilitated manner without the need for replacing the electrodes repeatedly. Furthermore, with such a high density array, a spatial filtering may be used to further improve the processing of the signals. For example, it may occur that two electrodes collect similar signals, then one of the signals may be disregarded, or the two signals may averaged to provide better single to noise ratio. Furthermore, blind signal separation may also be used for separation of signals within the set of signals provided by the plurality of electrodes. With a high density array, the individual electrode placement may be un-targeted, which means that they do not target a specific point on the surface of the limb. Instead, a control unit determines which electrodes collect useful data.

According to one embodiment of the invention, said plurality of electrodes may be arranged within a distance less than about 30 mm from each other, or less than about 20 mm from each, less than 10 mm or even less than 5 mm from each other.

According to one configuration, said high density electrode array is arranged around an entire outer circumference of said affected limb. Thus, the electrode array may be arranged in a collar arrangement placed around the limb. With a large surface coverage of the high density array, an enhanced signal quality may be obtained.

According to one embodiment of the invention, wherein said control unit may further be configured to:
determine, by feature selection or signal separation, which of said plurality of electrodes are collecting useful data, and
discard or process data collected from electrodes determined not to collect useful data. Useful data may be data in which a known characteristic is recognized. Thus, if an electrode appears to collect data with e.g. high levels of noise, the signal from that electrode may be determined to be disregarded by the control unit. Furthermore, the control unit may recognize from the collected data whether an electrode is loose (e.g. "lead-off") from the surface of the patent's skin. Feature selection relates to finding and selecting a set of features from the signals recorded by the electrode(s). "Brute force" algorithms or "Principal Component Analysis" can be used to select suitable sets of features which characterize better the bioelectric pattern of each movement found in the signal from an electrode.

According to one embodiment of the invention, further comprising a first and a second force applying element arranged to apply a respective first and second force to a respective first and second electrode in a direction towards a surface of said limb. The first and the second force may be maintained or changed in order to further improve the signal strength from the electrodes. The force applying means may be arranged on a stand with elongated first and second force applying elements directed towards respective electrodes. The patient may reach and adjust the force of the applied by the force applying elements in order to improve the signal from the respective electrode.

The electrodes may be implemented as a smart textile, which embeds dry electrodes with conductive leads paths to the control unit. This enables a simple and fast way of arranging the electrodes on the skin of the patient. For example, all the electrodes may be placed on the skin of the patient almost simultaneously by placing the smart textile with the electrodes in place on the patient on the limb.

Note that the embodiments mentioning specific electrode arrangements (e.g. high density array, force applying means, smart textile, and feature selection/signal separation) are applicable also independent from the other embodiments mentioned herein.

According to a second aspect of the invention, there is provided a method for controlling a system for neuromuscular rehabilitation of a patient having an affected limb, the method comprising the steps of: acquiring, via a plurality of electrodes, electric signals generated from a portion of the patient's body, the at least one electric signal corresponding to an intent to move the affected limb; performing pattern recognition of the electric signals; predicting motion intent in at least one joint using at least one feature in the electric signal, such that aggregated motions of the affected limb are predicted; and based on output signals from the performed pattern recognition, controlling a feedback member to perform actions corresponding to the motions, whereby the actions of the feedback member are individually and simultaneously controlled by the patient via the intended motions.

According to one embodiment of the invention, the feedback member is a virtual limb shown on a display for providing the visual feedback, wherein the actions are motions performed by the virtual limb, such that the method comprises: based on the output signals from the performed pattern recognition, controlling the virtual limb on the display to perform the motions, whereby the motions of the virtual limb are individually and simultaneously controlled by the patient via the intended motions.

According to one embodiment of the invention, the method comprises the step of superimposing the virtual limb onto a predetermined portion of the patient's body in the visual feedback being displayed on the display, the virtual limb corresponding to the affected limb.

According to one embodiment of the invention, the pattern recognition comprises the steps of: dividing each of the electric signals into signal segments defined by time windows; extracting signal features from at least one of the segments; combining the features into a feature vector; and based on the feature vector, predicting the intended motion of the affected limb, wherein the features comprise the cardinality of data elements of the electrical signal. Moreover, the cardinality may be used, exclusively, or in combination with other time and frequency domain features.

According to one embodiment of the invention, the method may comprise the steps of: executing predetermined motions predefined by the control unit; associating the features in the electric signals with the executed predetermined motions; performing rehabilitation tasks by the patient, wherein the tasks are training motions predetermined in the control unit; and reporting the patient's progress on the display. The training motion may for example be individual or simultaneous joint actuations (e.g. hand open/close). Reporting may be done in an electronic questionnaire presented on the display. Furthermore, reporting may be performed via predicted intended motions by the patient and may comprise a description by the patient on the experience of the performing the tasks, as well as the performance in the tasks. Reporting may for example comprise entering the amount of pain experienced by the patient during a predetermined time. For example, the progression of the pain may be reported. Furthermore, the prior to reporting the patient's progress, the control may predict the training motions based the associated features, wherein said feedback member performs said training motions.

Effects and features of this second aspect of the present invention are largely analogous to those described above in connection with the first aspect of the invention.

According to a third configuration there is provided a computer program product comprising a computer readable medium having stored thereon computer program means for a system for neuromuscular rehabilitation of a patient having an affected limb, the system comprising a control unit, wherein the computer program product comprises: code for acquiring, via a plurality of electrodes, electric signals generated from a portion of the patient's body, the at least one electric signal corresponding to an intent to move the affected limb; code for performing pattern recognition of the electric signals; code for predicting motion intent in at least one joint using at least one feature in the electric signal, such that aggregated motions of the affected limb are predicted; and code for, based on output signals from the performed pattern recognition, controlling a feedback member to perform actions corresponding to the motions, whereby the actions of the feedback member are individually and simultaneously controlled by the patient via the intended motions.

Effects and features of this third configuration are largely analogous to those described above in connection with the first and second aspects of the invention.

According to a fourth aspect of the present invention, there is provided a method for controlling an artificial limb comprising the steps of: acquiring an electrical signal generated from a portion of a patient's body; during acquisition, dividing the electric signals into signal segments defined by time windows; extracting signal features from at least one of the segments; forming a feature vector comprising the features; wherein the extracting features comprise at least the cardinality of a vector comprising data elements corresponding to the electrical signal in at least one segment.

A cardinality of a vector is the number of unique elements. For example, if a signal is defined by the elements in the vector [1 1 2 3 6] the cardinality of the signals is four. Using the cardinality of the signal in each segment as a feature for pattern recognition the accuracy of the pattern recognition is increased. The cardinality could for example be computed by sorting the data elements of a segment and delete repeated elements, then count the remaining data elements. The feature of cardinality has been realized to enhance the capability to predict individual and simultaneous motions.

According to one configuration, the artificial limb may be a prosthetic device. For example, a prosthetic device may be a prosthetic arm, leg, hand, foot, etc.

According to one configuration, the artificial limb may be a robotic device. For example, a remotely controlled vehicle, helicopter, or any other device which may be remotely controlled.

According to one configuration, the robotic device may be controlled by a patient via intended motions actuated by a joint adjacent to a limb of the patient to perform at least two actions.

According to one embodiment of the invention, the artificial limb may be a virtual limb displayed on a display. For example, the virtual limb may be part of a virtual reality or augmented reality shown to the patient on the display as a real-time visual feedback.

According to one embodiment of the invention, the electrical signals are acquired via surface or implantable electrodes. For example, implantable electrodes may be implantable into a patient's body and be arranged to connect with the nerves and/or muscles of the patient.

Effects and features of this fourth aspect of the present invention are largely analogous to those described above in connection with the previous aspects of the invention.

According to a fifth configuration, there is provided for use of a system for neuromuscular rehabilitation of a patient having an affected limb, said system comprising: a feedback member arranged to provide real-time visual feedback to said patient; a plurality of electrodes each arranged to acquire an electric signal generated from a portion of said patient's body, said at least one electric signal corresponding to an intent to move said affected limb; and a control unit configured to: perform pattern recognition of said electric signals, wherein at least one feature in said electric signal is used to predict motion intent of said affected limb adjacent to at least one joint, such that aggregated motions of said affected limb are predicted; and based on output signals from said performed pattern recognition, control said feedback member to perform actions corresponding to said aggregated motions, whereby said actions of said feedback member are individually and simultaneously controlled by said patient via said intended motions, wherein said use comprises:
executing predetermined motions predefined by said control unit; associating said features in said electric signals with said executed predetermined motions;
performing rehabilitation tasks by said patient, wherein said tasks are training motions predetermined in said control unit;
predicting said training motions based on the associated features and on the acquired signals from said portion of the patients body; wherein said feedback member performs said predicted training motions; and
reporting said patient's progress on said display.

Thus, first the patient executes motion such that the control unit may associate features in the signals with the motions. Next, the patient performs training motions. The control unit collects electric signals from the electrodes and based on the signals and on the associated features, the control unit predicts the training motions. The feedback member performs the predicted training motions. Reporting may be to report the progression of pain felt by the patient in the limb (e.g. phantom pain) or the overall repeatability of the predicted training motions (i.e. if the predicted training motions correspond with the performed motions). The reporting may be stored in the control unit for further processing of follow up b e.g. a medical expert.

Effects and features of this fifth configuration are largely analogous to those described above in connection with the previous aspects of the invention.

Further features of, and advantages with, the present invention will become apparent when studying the appended claims and the following description. The skilled person realize that different features of the present invention may be combined to create embodiments other than those described in the following, without departing from the scope of the present disclosure.

### BRIEF DESCRIPTION OF THE DRAWINGS

Fig. 1 illustrates a schematic drawing of a system according to an alternative embodiment of the invention;
Fig. 2 illustrates a schematic drawing of a system according to an alternative embodiment of the invention;
Fig. 3 illustrates exemplary individual motions of a limb;
Fig. 4 illustrates a flow-chart of an alternative embodiment according to the invention;
Fig. 5 illustrates a flow-chart of an alternative embodiment according to the invention; and
Fig. 6 illustrates an exemplary electrode placement;

### DESCRIPTION OF EXAMPLE EMBODIMENTS OF THE PRESENT INVENTION

In the following description, the present invention is mainly described with reference to neuromuscular rehabilitation in relation to phantom limb pain in a patient with a missing limb. It should, however, be noted that this by no means limits the scope of the invention, which is equally applicable to neuromuscular rehabilitation of any other condition where myoelectric and/or neuroelectric signals can be used to drive the system and promote motor execution. For example, such as neuromuscular rehabilitation of a patient suffering from e.g. stroke, incomplete spinal cord, and/or nerve injuries.

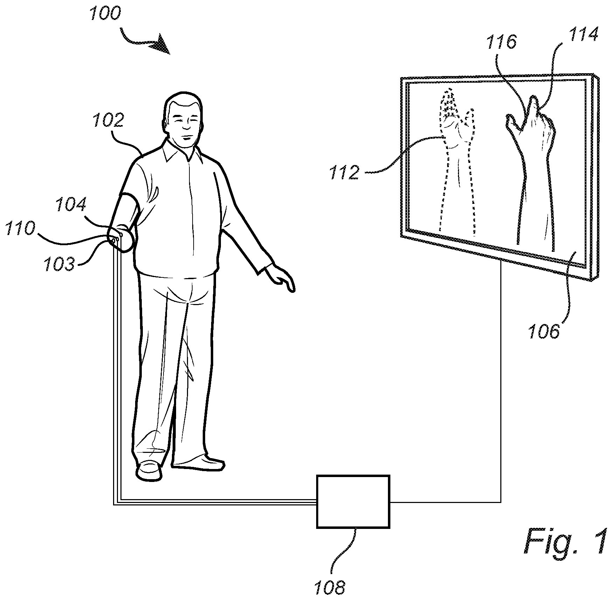

Fig. 1 illustrates a schematic of a system 100 according to an exemplary embodiment of the invention. Fig. 1 shows a patient 102 having an affected limb 104, the patient 102 is using the system 100. In this case the affected limb 104 has a missing portion such that there is missing limb, for example as a result of an amputation. Furthermore, Fig. 1 shows a display 106 connected to a control unit 108 and a plurality of electrodes 110 also connected to the control unit 108. Although three electrodes are shown in Fig. 1, any suitable number of electrodes is possible to use. The connection between the display 106 and the control unit 108 may be enabled through physical wires or by wireless connection. The electrodes 110 are arranged to acquire an electric signal generated from a portion 103 of the patient's body, in this particular case the stump 103 of a limb having a missing portion. As illustrated in this exemplary embodiment, the electrodes 110 are attached on the surface of the patient's skin such that myoelectric signals may be recorded from the patient. However, the electrodes 110 may also be implantable electrodes 110. The control unit 108 is configured to read electric signals acquired by the electrodes 110 and perform pattern recognition of the electric signals. The control unit 108 processes the electric signals and recognizes features in the electric signals such that motion intent of aggregated motions is predicted. In other words, the control unit 108 predicts motions of the missing limb intended by the patient 102. After the intended motion has been predicted, the control unit 108 controls the virtual limb 116 on the display to perform the intended motions. For example, if a patient 102 intends to move his limb at a first j oint, for example the elbow, and at the same time supinate the hand such that it rotates, the virtual limb 116 will move from a first position 112 to a second position 114 according to the aggregated intended motions. In the first position the missing limb is to the left and the palm of the hand faces the patient 102 viewing the display, then the aggregated motion of the limb intended by the patient 102 is to flex the elbow joint and to pronate the hand, the intended motion is predicted by the control unit 108 and the limb moves to position 114. Other motions are of course possible such as for example flexing of a limb, extending the limb, pronation, supination, open or close hand (in the case where a hand is part of an affected limb), or any other motion possible with a limb, and/or combinations thereof. The control unit 108 may be connected to the display 106 by a wireless connection or via physical electrical connections. Moreover, although in Fig.1 a virtual arm is shown, a robotic device may be used instead. For example, the display with the virtual arm may be replaced by a prosthetic device or a remotely controlled vehicle or similar.

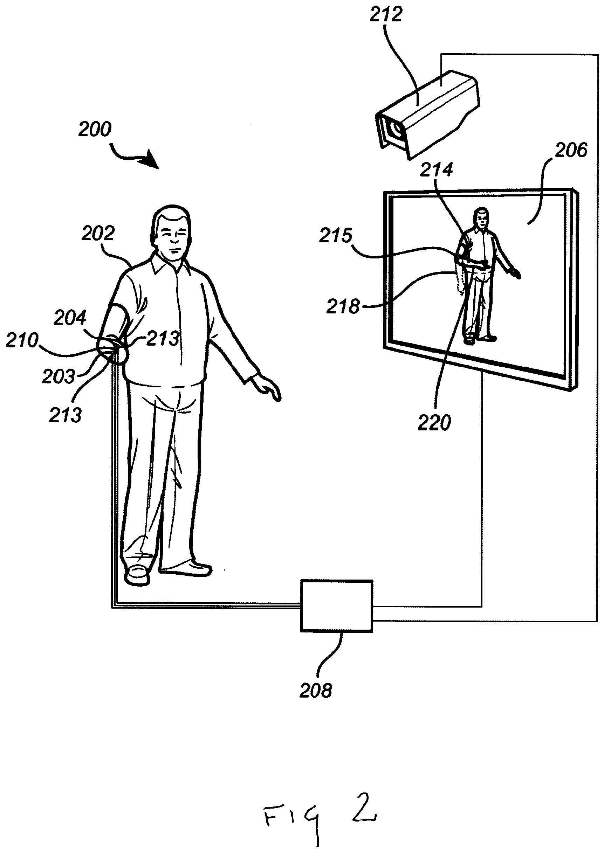

Fig. 2 shows another system 200 according to an embodiment of the invention. Similar to what is depicted in Fig. 1, Fig. 2 shows a patient 202 having an amputated limb 204, and the patient 202 is using the system 200. At the stump 203 of the amputated limb there is a plurality of electrodes 210 arranged to acquire an electric signal at the stump 203. Furthermore, the electrodes 210 are connected to a control unit 208 and there is also a display 206 connected to the control unit 208. Although three electrodes are shown in Fig. 2, any suitable number of electrodes is possible. The display 206 is configured to provide real-time feedback to the patient 202 and to show a virtual limb 214. Furthermore, there is a video capturing device in the form of a camera 212 arranged to acquire a video stream of the patient 202. The camera 212 may be a conventional webcam. For example the camera 212 may be a simple traditional webcam. Moreover, the video stream is shown on the display 206. In other words the patient 202 can see him/herself in real-time on the display 206. The control unit 208 is configured to superimpose the virtual limb 214 onto a predetermined portion 215 of the patient's body shown on the display 206. As shown in Fig. 2, the virtual limb 214 is a portion of an arm and the hand of the arm, superimposed onto the stump 215 of the affected limb being an amputated limb. According to the invention, there are markers 213 such as fiducial markers 213 arranged on the stump of the amputated limb on the patient such that the control unit 208 may track the motion of the affected limb 204 via the webcam 212. This enables the virtual limb 214 to be aligned anatomically correctly with the stump 215 of the amputated limb of the patient 216 on the display. Moreover, if the patient 202 moves around, the virtual limb 214 stays in the anatomically correct position on the display 206. Similar to what is described with reference to Fig. 1, the control unit 208 is arranged to read electric signals acquired by the electrodes 210 and to perform pattern recognition on the electric signals. The control unit 208 processes the electric signals and recognizes features in the electric signals such that motion intent of aggregated motions is predicted. In other words, the control unit 208 predicts aggregated motions of the missing limb intended by the patient. After the intended motions have been predicted, the control unit 208 controls the virtual limb 214 on the display to perform the intended motions. For example, if a patient intends to move his limb at a first j oint, for example the elbow, and at the same time supinate the hand such that it rotates, the virtual limb will move from a first position 218 to a second position 220 according to the intended motion. In the first position 218 the missing limb is to the left and the palm of the hand faces away from the patient 202 viewing the display 206, then the motion of the limb intended by the patient is to flex the elbow joint and to supinate the hand, the intended motion is predicted by the control unit 208 and the virtual limb moves to position 220. Other motions are of course possible, such as for example flexing of a limb, extending the limb, pronation, supination, open or close hand (in the case where a hand is part of an affected limb), or any other motion possible with a limb, and/or combinations thereof. The control unit 208 may be connected to the display 206 by a wireless connection or via physical electrical connections.

Superimposing of the virtual limb onto a predetermined portion of the patient's body as described with reference to Fig. 2 enables an augmented reality. Furthermore, the patient may play a video game shown on the display via intended motions. In other words, the conventional mouse and keyboard are substituted by predicted motions.

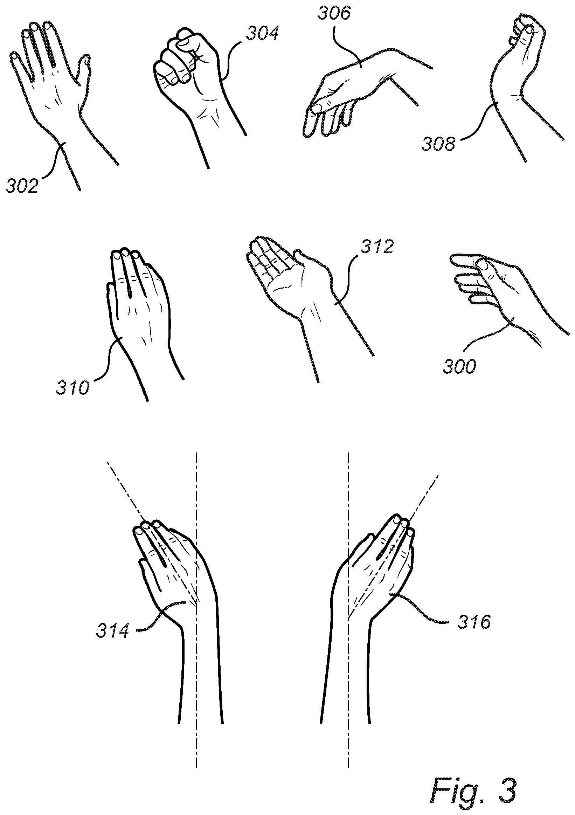

Fig. 3 illustrates exemplary individual motions of a limb. In this case the limb is a hand, however, the limb may within the context of the invention be any limb such as an arm, a leg, a foot, etc. In Fig. 3 a limb, illustrated as a hand, is shown in a resting position 300, and the other positions 302-316 are relative to the resting position 300. The other positions are: open hand 302, closed hand 304, flexed position 306, extended position 308, pronation 310, supination 312, ulnar deviation 314, and radial deviation 316. Note that other motions are also possible. For example, it is possible to move the hand in any of the above motions at the same time as performing any motion with the arm adjacent to the elbow.

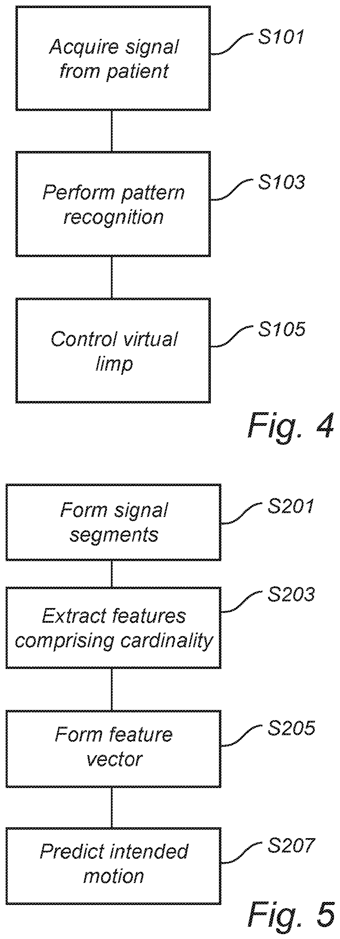

Fig. 4 is a flow-chart illustrating the method steps according to an embodiment of the invention. The method may be implemented using for example the system depicted in Fig. 1 or Fig. 2. In a first step S101 electrical signals are acquired from a patient. The electrical signals may for example be acquired from surface electrodes mounted on the surface of the skin of the patient or may be acquired from electrodes implanted into the patient and connected to nerves of the patient. In a second step S103, pattern recognition is performed on the electrical signals acquired by the electrodes. During pattern recognition, features of the electric signals are distinguished and determined to correspond to certain motions of the limb intended by the patient. In a final step S105, a virtual limb is controlled to perform motions based on the predicted motions. The virtual limb is displayed on a display shown to the patient. Optionally, a robotic device is controlled via the pattern recognition, for example a prosthetic device may be controlled.

Fig. 5 is a flow-chart illustrating the method steps according to another embodiment of the invention. In Fig. 5 the pattern recognition S 103 steps are illustrated in more detail. In a first step S201, the electrical signals acquired from the patient are divided into segments. The length of the segments is defined in terms of predetermined time windows. For example, a time window may have 200 ms duration. Moreover, each electrical signal from the independent electrodes is divided into segments. Within each segment at least one characteristic features is extracted. A non-exhaustive list is of features of in the time domain of an electrical signal is comprised in the following: mean absolute value, median, standard deviation, variance, waveform length, root mean square (RMS), zero-crossings of the signal, peaks (RMS), peaks mean, mean velocity, slope changes, power, difference absolute mean, max fractal length, fractal dimension, fractal dimension Higuchi, rough entropy, correlation, co-variance, etc. A non-exhaustive list is of features in the frequency domain of an electrical signal is comprised in the following: waveform length, mean, median, peaks mean, peaks median, peaks standard deviation, etc. Furthermore, the cardinality of the segments is extracted as a characteristic feature. The cardinality of a signal is the number of unique elements of the signal. For example, if a signal is defined by the elements in the vector [1 1 2 3 5] the cardinality of the signals is four. Using the cardinality of the signal in each segment as a feature for pattern recognition, the accuracy of the pattern recognition is increased. As an example, the following is the average class-specific accuracy of a commonly used classifier (Linear Discriminant Analysis, LDA) in the prediction of 11 hand and wrist motions in 20 subjects when fed with the following features:
Cardinality: 90.0%
Zero crossings: 82.1%
Wave length: 82.0%
Root mean square value: 81.8%
Mean absolute value: 80.1%
Sign slope change: 74.7%

As can be seen from the above accuracies of prediction, the prediction accuracy when using the cardinality feature is higher compared to when using the other commonly used features listed above, resulting in an improved pattern recognition and thus prediction of intended motions.

In a subsequent step S205, a feature vector is formed by placing the extracted features, including the cardinality, as entries in a vector for the present segment. From the feature vector, an intended motion is predicted S207 using known algorithms. Algorithms used for pattern recognition may be e.g. supervised, unsupervised, and/or competitive, as well of a statistical nature (e.g. LDA), or biologically inspired (e.g. artificial neural networks, ANN). The algorithms may be arranged in different topologies (e.g. One-Vs.-One).

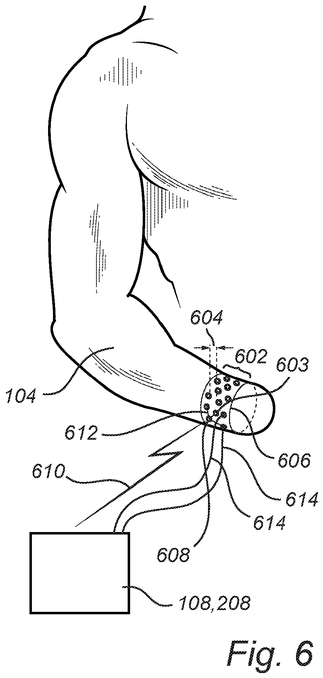

Fig. 6 illustrates an array of electrodes arranged on a limb of a person, for example the limb may be an affected limb 104. On the skin of the limb there is a high density array 602 of electrodes 603 (only one is numbered). The number of electrodes is at least six, but may be for example, 8, 10, 12 or more. The electrodes 603 are arranged close to each other in order to densely cover a large area of the limb. For example, the distance 604 between adjacent electrodes may be less than 30 mm, or even less such as less than 10 mm or less than 5 mm. With such a high density array 602, the placement of the individual electrodes 603 is less crucial, instead a control unit 108, 208, 608 may determine which signals are useful. The control unit 608 may be arranged adjacent to the electrodes 603 and may communicate wirelessly 610 with the control unit 108, 208. Alternatively, or additionally the control unit 108, 208 communicates directly with the electrodes via cables 614. In one example embodiment, the array 602 of electrodes extends around the outer circumference 606 of the limb in order to cover a large area around the limb 104. The control unit 108, 208, or 608 may determine from the signals acquired from the electrodes 603 of the array 602 which of the signals are useful. For example, the control unit 108, 208, or 608 may use a feature selection algorithm or a signal separation algorithm to determine if a signal is useful. The electrode arrangement embodiment shown in Fig. 6 may be used separate from the other embodiments described herein.

Additionally, variations to the disclosed embodiments can be understood and effected by the skilled person in practicing the claimed invention, from a study of the drawings, the disclosure, and the appended claims. For example, virtual reality is also possible within the context of the invention although only augmented reality is shown in the exemplary embodiment of Fig. 2. The system and method may further be used for controlling a robotic device such as a remotely controlled toy vehicle, helicopter, boat, robotic arm such as for example a prosthesis, etc. Furthermore, although three electrodes are shown in Fig. 1 and Fig. 2, any suitable number of electrodes is possible within the scope of the invention.

Furthermore, the methods disclosed and described may be used by a person not having an affected limb, thus the method and the system is equally applicable to a person having only non-affected limbs.

Furthermore, the communication between electrodes and the control unit may be either via wires of via wireless communication. In case of wireless communication, there may be a second control unit arranged nearby the electrodes.

The invention is defined by the appended claims.

## Claims

1. A system (100, 200) for neuromuscular phantom limb pain rehabilitation of a patient (102, 202) having an affected limb (104, 204), said system (100, 200) comprising:
a control unit;
a video capturing device connected to the control unit and arranged to acquire a video stream of said patient (102, 202), and to provide the video stream to the control unit,
a display (106, 206) connected to the control unit, and arranged to receive the video stream from the control unit and provide real-time visual feedback comprising the video stream and a virtual limb (214) to said patient (102, 202),
a plurality of electrodes (110, 210) connected to the control unit and each configured to provide, to the control unit, at least one electric signal generated from a portion of said patient's (102, 202) body, said at least one electric signal corresponding to an intent to move said affected limb (104, 204); and
wherein the control unit (108, 208) is configured to:
perform pattern recognition of said electric signals, wherein features in said electric signals is used by the control unit to predict motion intent of said affected limb (104, 204) adjacent to at least one joint, to thereby predict aggregated motions being individual motions of several joints of said affected limb (104, 204) performed at least partly simultaneously;
track a predetermined portion of said patient's (102, 202) body in the video stream, based on tracking of fiducial markers (213) arranged on the affected limb (104, 204);
in said real-time real time visual feedback, superimpose said virtual limb (214) onto the predetermined portion of said patient's (102, 202) body, and control said virtual limb to follow said predetermined portion of said patient's (102, 202) body in said real-time visual feedback such that said virtual limb (214) remains in an anatomically correct position,
based on output signals from said performed pattern recognition, control said virtual limb on said display (106, 206) to perform motions corresponding to said aggregated motions, whereby said motions of said virtual limb are individually and simultaneously controlled by said patient (102, 202) via said intended motions.

2. The system (100, 200) according to claim 1, wherein at least two aggregated motions of said affected limb (104, 204) are predicted, wherein said control unit (108, 208) is configured to control said virtual limb (116, 214) to perform at least two actions corresponding to said at least two motions.

3. The system (100, 200) according to any of the preceding claims, wherein, for pattern recognition, said control unit (108, 208) is configured to:
divide each of said electric signals into signal segments defined by time intervals;
extract signal features from at least one of said segments;
combine said features into a feature vector relating to said motion; and
based on said feature vector, predict said intended motion of said affected limb,
wherein said features comprise an extracted cardinality of the data elements of said electrical signal.

4. The system (100, 200) according to any of the preceding claims, wherein said control unit (108, 208) is configured to, based on said output signals from said pattern recognition, control a video game on said display (106, 206).

5. The system (100, 200) according to any of the preceding claims, wherein said electrodes (110, 210) are implantable into a patient's (102, 202) body for detecting bioelectrical signals.

6. The system (100, 200) according to claim 1, wherein said control unit (108, 208) is configured to, based on said output signals from said pattern recognition, control computer unit input commands.

7. The system (100, 200) according to any of claims 1 to 6, wherein said plurality of electrodes is a high density electrode array (602).

8. The system (100, 200) according to claim 7, wherein said plurality of electrodes are arranged within a distance (604) less than 30 mm of each other, preferably less than 15 mm from each other.

9. The system (100, 200) according to any of the preceding claims, wherein said control unit (108, 208, 608) is further configured to:
determine, by feature selection or signal separation, which of said plurality of electrodes are collecting useful data, and
discard or process data collected from electrodes determined not to collect useful data.

10. The system (100, 200) according to any of the preceding claims, further comprising a first and a second force applying element arranged to apply a respective force to a respective first and second electrode in a direction towards a surface of said limb.

11. A method for controlling a system (100, 200) for neuromuscular phantom limb pain rehabilitation by using visual feedback comprising a virtual limb shown on a display, said method comprising the steps of:
acquiring a video stream of a patient (102, 202) comprising a person's limb (104, 204) for providing the visual feedback,
tracking a predetermined portion of said patient's (102, 202) body in the video stream based on tracking of fiducial markers (213) arranged on the affected limb (104, 204);
superimposing a virtual limb onto the predetermined portion of said person's (102, 202) body in said visual feedback being displayed on said display (106, 206), said virtual limb corresponding to said limb (104, 204), wherein said virtual limb (214) follows said predetermined portion of said patient's (102, 202) body in said visual feedback such that said virtual limb (214) remains in an anatomically correct position,
acquiring (S101), via a plurality of electrodes (110, 210), electric signals generated from a portion of said person's (102, 202) body, said at least one electric signal corresponding to an intent to move said limb (104, 204);
performing (S103) pattern recognition of said electric signals;
predicting motion intent in several joints using at least one feature in said electric signals, such that aggregated motions being individual motions of several joints of said limb (104, 204) performed at least partly simultaneously are predicted; and
based on output signals from said performed pattern recognition, controlling (S105) said virtual limb on said display to perform motions corresponding to said aggregated motions, whereby said motions of said virtual limb are individually and simultaneously controlled by said person (102, 202) via said intended motions.

## Patentansprüche

1. System (100, 200) zur neuromuskulären Phantomschmerzen-Rehabilitation von einem Patienten (102, 202) mit einem betroffenen Glied (104, 204), wobei das System (100, 200) umfasst:
eine Steuereinheit;
ein Videoaufnahmegerät, das mit der Steuereinheit verbunden ist und dazu dient, einen Videostream des Patienten (102, 202) zu erfassen und den Videostream der Steuereinheit bereitzustellen,
ein Display, das mit der Steuereinheit Display (106, 206) verbunden ist, und angeordnet ist, um den Videostream von der Steuereinheit zu empfangen und dem Patienten (102, 202) visuelles Echtzeit-Feedback zu liefern, das den Videostream und ein virtuelles Glied (214) umfasst,
eine Vielzahl von Elektroden (110, 210), die mit der Steuereinheit verbunden und jeweils so konfiguriert sind, dass sie der Steuereinheit mindestens ein elektrisches Signal liefern, das von einem Teil des Körpers des Patienten (102, 202) erzeugt wird, wobei das mindestens eine elektrische Signal entsprechend einer Absicht, das betroffene Glied (104, 204) zu bewegen, verwendet wird; und
wobei die Steuereinheit (108, 208) für Folgendes konfiguriert ist:
Durchführen einer Mustererkennung der elektrischen Signale, wobei Merkmale in den elektrischen Signalen von der Steuereinheit verwendet werden, um die Bewegungsabsicht des betroffenen Glieds (104, 204) neben mindestens einem Gelenk vorherzusagen, um dadurch aggregierte Bewegungen vorherzusagen, die einzelne Bewegungen mehrerer Gelenke des betroffenen Gliedes (104, 204) sind, die zumindest teilweise gleichzeitig ausgeführt werden;
Verfolgen eines vorbestimmten Teils des Körpers des Patienten (102, 202) im Videostream basierend auf der Verfolgung von Referenzmarkierungen (213), die auf dem betroffenen Glied (104, 204) angeordnet sind;
Überlagern in dem visuellen Echtzeit-Feedback des virtuellen Glieds (214) dem vorgegebenen Teil des Körpers des Patienten (102, 202) und Steuern des virtuellen Glieds derart, dass es dem vorgegebenen Teil des Körpers des Patienten (102, 202) in dem visuellen Echtzeit-Feedback derart folgt, dass das virtuelle Glied (214) in einer anatomisch korrekten Position bleibt,
Steuern des virtuellen Glieds auf der Anzeige (106, 206) basierend auf Ausgangssignalen der durchgeführten Mustererkennung, um Bewegungen auszuführen, die den aggregierten Bewegungen entsprechen, wobei die Bewegungen des virtuellen Glieds einzeln und gleichzeitig vom Patienten (102, 202) über die vorgesehenen Bewegungen gesteuert werden.

2. System (100, 200) nach Anspruch 1, wobei mindestens zwei aggregierte Bewegungen des betroffenen Glieds (104, 204) vorhergesagt werden, wobei die Steuereinheit (108, 208) derart konfiguriert ist, dass sie das virtuelle Glied (116, 214) derart steuert, dass es mindestens zwei Aktionen ausführt, die mindestens zwei Bewegungen entsprechen.

3. System (100, 200) nach einem der vorhergehenden Ansprüche, wobei, zur Mustererkennung, die Steuereinheit (108, 208) für Folgendes konfiguriert ist:
Aufteilen jedes der elektrischen Signale in durch Zeitintervalle definierte Signalsegmente;
Extrahieren von Signalmerkmalen aus mindestens einem der Segmente;
Kombinieren der Merkmale zu einem Merkmalsvektor, der sich auf die Bewegung bezieht; und
basierend auf dem Merkmalsvektor, Vorhersagen der beabsichtigten Bewegung des betroffenen Gliedes,
wobei die Merkmale eine extrahierte Kardinalität der Datenelemente des elektrischen Signals umfassen.

4. System (100, 200) nach einem der vorhergehenden Ansprüche, wobei die Steuereinheit (108, 208) dazu konfiguriert ist, basierend auf den Ausgangssignalen der Mustererkennung ein Videospiel auf dem Display (106, 206) zu steuern.

5. System (100, 200) nach einem der vorhergehenden Ansprüche, wobei die Elektroden (110, 210) in den Körper eines Patienten (102, 202) implantierbar sind, um bioelektrische Signale zu erfassen.

6. System (100, 200) nach Anspruch 1, wobei die Steuereinheit (108, 208) derart konfiguriert ist, dass sie basierend auf den Ausgangssignalen der Mustererkennung Eingabebefehle der Computereinheit steuert.

7. System (100, 200) nach einem der Ansprüche 1 bis 6, wobei die Vielzahl von Elektroden ein hochdichtes Elektrodenarray (602) ist.

8. System (100, 200) nach Anspruch 7, wobei die Mehrzahl von Elektroden in einem Abstand (604) von weniger als 30 mm voneinander, vorzugsweise weniger als 15 mm voneinander, angeordnet sind.

9. System (100, 200) nach einem der vorhergehenden Ansprüche, wobei die Steuereinheit (108, 208, 608) weiterhin für Folgendes konfiguriert ist:
Bestimmen durch Merkmalsauswahl oder Signaltrennung, welche der mehreren Elektroden nützliche Daten sammeln, und
Verwerfen oder verarbeiten von Daten, die von Elektroden gesammelt wurden, bei denen festgestellt wurde, dass sie keine nützlichen Daten sammeln.

10. System (100, 200) nach einem der vorhergehenden Ansprüche, ferner umfassend ein erstes und ein zweites Kraft ausübendes Element, die so angeordnet sind, dass sie eine entsprechende Kraft auf eine entsprechende erste und zweite Elektrode in einer Richtung zu einer Oberfläche des Gliedes ausüben.

11. Verfahren zur Steuerung eines Systems (100, 200) für neuromuskuläre Phantomglied-Schmerzrehabilitation durch visuelles Feedback, bestehend aus einem virtuellen Glied, das auf einem Display angezeigt wird, wobei das Verfahren die folgenden Schritte umfasst:
Erfassen eines Videostreams eines Patienten (102, 202), der ein Glied einer Person (104, 204) umfasst, zur Bereitstellung des visuellen Feedbacks,
Verfolgen eines vorbestimmten Teils des Körpers des Patienten (102, 202) im Videostream basierend auf der Verfolgung von Referenzmarkierungen (213), die auf dem betroffenen Glied (104, 204) angeordnet sind;
Überlagern eines virtuellen Glieds mit dem vorbestimmten Teil des Körpers der Person (102, 202) in dem visuellen Feedback, das auf dem Display (106, 206) angezeigt wird, wobei das virtuelle Glied dem Glied (104, 204) entspricht, wobei das virtuelle Glied (214) dem vorbestimmten Teil des Körpers des Patienten (102, 202) in dem visuellen Feedback folgt, so dass das virtuelle Glied (214) in einer anatomisch korrekten Position bleibt,
Erfassen (S101), über mehrere Elektroden (110, 210), elektrischer Signale, die von einem Teil des Körpers der Person (102, 202) erzeugt werden, wobei das mindestens eine elektrische Signal einer Absicht entspricht, das Glied (104, 204) zu bewegen;
Durchführen (S103) einer Mustererkennung der elektrischen Signale;
Vorhersage der Bewegungsabsicht in mehreren Gelenken unter Verwendung mindestens eines Merkmals in den elektrischen Signalen, so dass aggregierte Bewegungen vorhergesagt werden, bei denen es sich um einzelne Bewegungen mehrerer Gelenke des Glieds (104, 204) handelt, die zumindest teilweise gleichzeitig ausgeführt werden; und
Steuern (S105) des virtuellen Glieds auf der Anzeige, basierend auf Ausgangssignalen der durchgeführten Mustererkennung, um Bewegungen auszuführen, die den aggregierten Bewegungen entsprechen, wobei die Bewegungen des virtuellen Glieds einzeln und gleichzeitig von der Person (102, 202) über besagte beabsichtigte Bewegungen gesteuert werden.

## Revendications

1. Système (100, 200) de rééducation neuromusculaire dans les douleurs fantômes des membres d'un patient (102, 202) ayant un membre affecté (104, 204), ledit système (100, 200) comprenant :
une unité de commande ;
un dispositif de capture vidéo connecté à l'unité de commande et agencé pour acquérir un flux vidéo dudit patient (102, 202), et pour fournir le flux vidéo à l'unité de commande,
un écran (106, 206) relié à l'unité de commande, et agencé pour recevoir le flux vidéo de l'unité de commande et fournir un retour visuel en temps réel comprenant le flux vidéo et un membre virtuel (214) audit patient (102, 202),
une pluralité d'électrodes (110, 210) connectées à l'unité centrale et chacune configurée pour fournir, à l'unité de commande, au moins un signal électrique généré d'une partie du corps dudit patient (102, 202), l'au moins un signal électrique correspondant à une intention de bouger ledit membre affecté (104, 204) ; et
dans lequel l'unité de commande (108, 208) est configurée pour :
effectuer la reconnaissance de modèle desdits signaux électriques, dans lequel des caractéristiques dans lesdits signaux électriques sont utilisées par l'unité de commande pour prédire l'intention de mouvement dudit membre affecté (104, 204) adjacent à l'au moins une articulation, pour prédire ainsi des mouvements agrégés comme étant des mouvements individuels de plusieurs articulations dudit membre affecté (104, 204) réalisés au moins partiellement simultanément ;
suivre une partie prédéterminée du corps dudit patient (102, 202) dans le flux vidéo, en se basant sur le suivi de repères (213) agencés sur le membre affecté (104, 204) ;
dans le retour visuel en temps réel, superposer ledit membre virtuel (214) sur la partie prédéterminée du corps dudit patient (102, 202), et commander ledit membre virtuel à suivre ladite partie prédéterminée du corps dudit patient (102, 202) dans ledit retour visuel en temps réel de telle façon que ledit membre virtuel (214) reste dans une position anatomiquement correcte,
en se basant sur des signaux en sortie de ladite reconnaissance de modèle effectuée, commander ledit membre virtuel sur ledit écran (106, 206) à exécuter des mouvements correspondant auxdits mouvements agrégés, ce par quoi lesdits mouvements dudit membre virtuel sont commandés individuellement et simultanément par ledit patient (102, 202) par le biais des mouvements intentionnels.

2. Système (100, 200) selon la revendication 1, dans lequel au moins deux mouvements agrégés dudit membre affecté (104, 204) sont prédits, dans lequel ladite unité de commande (108, 208) est configurée pour commander ledit membre virtuel (116, 214) à exécuter au moins deux actions correspondant auxdits au moins deux mouvements.

3. Système (100, 200) selon l'une quelconque des revendications précédentes, dans lequel, pour la reconnaissance de modèle, ladite unité de commande (108, 208) est configurée pour :
diviser chacun desdits signaux électriques en segments de signal définis par des intervalles temporels ;
extraire les caractéristiques de signal d'au moins un desdits segments ;
combiner lesdites caractéristiques en un vecteur de caractéristiques relatif audit mouvement ; et
en se basant sur ledit vecteur de caractéristiques, prédire ledit mouvement intentionnel dudit membre affecté,
dans lequel lesdites caractéristiques comprennent une cardinalité extraite des éléments de données dudit signal électrique.

4. Système (100, 200) selon l'une quelconque des revendications précédentes, dans lequel ladite unité de commande (108, 208) est configurée pour, en se basant sur lesdits signaux de sortie de ladite reconnaissance de modèle, commander un jeu vidéo sur ledit écran (106, 206) .

5. Système (100, 200) selon l'une quelconque des revendications précédentes, dans lequel lesdites électrodes (110, 210) sont implantables dans le corps d'un patient (102, 202) pour détecter des signaux bioélectriques.

6. Système (100, 200) selon la revendication 1, dans lequel ladite unité de commande (108, 208) est configurée pour, en se basant sur lesdits signaux de sortie de ladite reconnaissance de modèle, commander des commandes de saisie d'unité d'ordinateur.

7. Système (100, 200) selon l'une quelconque des revendications 1 à 6, dans lequel ladite pluralité d'électrodes est une rangée d'électrodes haute densité (602) .

8. Système (100, 200) selon la revendication 7, dans lequel ladite pluralité d'électrodes sont agencées à une distance (604) inférieure à 30 mm l'une de l'autre, de préférence inférieure à 15 mm l'une de l'autre.

9. Système (100, 200) selon l'une quelconque des revendications précédentes, dans lequel ladite unité de commande (108, 208, 608) est en outre configurée pour :
déterminer, par sélection de caractéristiques ou séparation de signal, laquelle de ladite pluralité d'électrodes sont des données de collecte utiles, et
écarter ou traiter les données collectées des électrodes déterminées comme[1] n'étant pas des données de collecte utiles.

10. Système (100, 200) selon l'une quelconque des revendications précédentes, comprenant en outre un premier et un second élément applicateur de force pour appliquer une force respective sur une première et une seconde électrodes respectives dans une direction vers une surface dudit membre.

11. Procédé de commande d'un système (100, 200) pour la rééducation neuromusculaire dans les douleurs fantômes des membres, comprenant un membre virtuel montré sur un écran, ledit procédé comprenant les étapes de :
acquérir un flux vidéo d'un patient (102, 202) comprenant un membre (104, 204) de personne pour fournir le retour visuel,
suivre une partie prédéterminée du corps dudit patient (102, 202) dans le flux vidéo, en se basant sur le suivi de repères (213) agencés sur le membre affecté (104, 204) ;
superposer un membre virtuel sur ladite partie prédéterminée du corps dudit patient (102, 202) dans ledit retour visuel en temps réel affiché sur ledit écran (106, 206), ledit membre virtuel correspondant audit membre (104, 204), dans lequel ledit membre virtuel (214) suit ladite partie prédéterminée du corps dudit patient (102, 202) dans ledit retour visuel de telle façon que ledit membre virtuel (214) reste dans une position anatomiquement correcte,
acquérir (S101), par le biais d'une pluralité d'électrodes (110, 210), des signaux électriques générés d'une partie du corps de ladite personne (102, 202), ledit au moins un signal électrique correspondant à une intention de bouger ledit membre (104, 204) ;
réaliser (S103) une reconnaissance de modèle desdits signaux électriques ;
prédire l'intention de mouvement dans différentes articulations en utilisant au moins une caractéristique dans lesdits signaux électriques, de telle façon que des mouvements agrégés comme étant des mouvements individuels de différentes articulations dudit membre (104, 204) réalisés au moins partiellement simultanément sont prédits ; et
en se basant sur des signaux en sortie de ladite reconnaissance de modèle effectuée, commander (S105) ledit membre virtuel sur ledit écran à effectuer des mouvements correspondants auxdits mouvements agrégés, ce par quoi lesdits mouvements dudit membre virtuel sont commandés individuellement et simultanément par ladite personne (102, 202) par le biais desdits mouvements intentionnels.
